# EUROPEAN PATENT APPLICATION

(11) **EP 1 495 770 A1**
(43) Date of publication of application: **12.01.2005**
(21) Application number: 04388042.6
(22) Date of filing: 01.07.2004
(51) Int. Cl.: A61L 2/18, A61L 2/24

(54) **Device and method for sterilizing**

(30) Priority: 11.07.2003 DK 200301061
(71) Applicant: Slagteriernes Forskningsinstitut, 4000 Roskilde (DK)
(72) Inventor: Theilmann, Jens, 4000 Roskilde (DK); Moeller, Helge, 2920 Charlottenlund (DK)
(74) Representative: Nielsen, Kim Garsdal

(57) **Abstract**

A method and device as well as use thereof for sterilization of implements. A cycle is applied comprising a first spraying with cold water, a spraying with sterilizing fluid and a second spraying with cold water. The cold water and the sterilizing fluid are sprayed onto the implements through different nozzles (7, 8).

## Description

The present invention relates to a method for sterilization of implements, particularly knives, in a sterilization device which applies a cycle comprising a spraying with cold water and a spraying with sterilizing fluid, the cold water and the sterilizing fluid being sprayed onto the implements through nozzles, a device for sterilization of implements, particularly knives, and comprising nozzles for spraying cold water and sterilizing fluid onto the implements, as well as a device for sterilization of implements, particularly knives, and comprising a substantially closed housing with an insertion opening for at least one implement to be sterilized, holding members for the implement to be sterilized being arranged in connection with the insertion opening.

Slaughterhouses often apply a slaughterline speed of 400 carcasses per hour or more. At manual processing of the carcasses certain steps of the slaughtering process are subject to a requirement of sterilization of the knives between each carcass, and accordingly a fast and efficient method of sterilization is needed.

The Dutch company Nawi markets a device, MESSENSTERILISATOR ECONOMIC, TYPE 01177000, with a housing into which the knife is passed through an insertion opening for sterilization. In the housing a number of nozzles are arranged which spray hot water at 82°C onto the knife, whereby it is sterilized.

The use of water of at least 82°C is a requirement in the EU to ensure the sterilization. Alternatively, approved sterilizing fluids may be applied at lower temperatures.

The high temperature of the fluid, however, results in burnt deposits on the knife because the proteins from blood, for example, left on the knife after slaughtering denature and burn onto the knife at the prescribed temperature. Moreover, further burnt deposits may occur when the sterilized, still hot knife is used immediately for a new work operation, as proteins from the new carcass get in contact with the hot blade and also denature. The burnt deposits form a rough coating on the blade, considerably reducing the cutting ability of the knife, which is not desirable.

FR-A-2,751,880 describes a device comprising nozzles for sterilization of implements, which in addition to rinsing with sterilizing fluid also applies prerinsing and postrinsing with cold water. The purpose of such prerinsing and postrinsing is not mentioned, but it must be assumed that the prerinsing cleans blood, etc., off the implement, whereupon the sterilizing fluid sterilizes the knife, and the postrinsing cools the knife so that burnt deposits are prevented both at the sterilization and at the subsequent use of the knife on a new carcass.

This apparatus furthermore has the disadvantage that the sterilization process is too long for application at the above-mentioned slaughterline speed, and that it requires active user activation to initiate the sterilization process.

Furthermore, SFK Meat Systems, Denmark, markets a knife sterilization device comprising a housing with two doors coupled so that when one door closes the other one opens. On the inside of each door a holder is mounted for a knife to be sterilized. When the door closes, sterilization commences by spraying with water through nozzles. This opening and closing of doors as well as the placing of the knife in the holder are laborious. Moreover, the implement is hanging in the holders by means of the haft or handle in such a way that the holders screen off the upper parts of the blade from the sterilizing fluid at the transition of the blade to the haft so that sterilization of these parts is not completely ensured. This device also applies prerinsing and postrinsing at lower temperatures to prevent burnt deposits.

Both devices mentioned above furthermore have the disadvantage that the same nozzles are applied for both prerinsing, sterilization and postrinsing. This delays the sterilization process because, at each shift from cold to hot water, or vice versa, the nozzles and the connected pipe system have to be heated or cooled, respectively, by the hot or cold water before the water takes effect. For effective sterilization to be performed, extra time for these temperature shifts must be anticipated.

The object of the present invention is to provide a sterilization device and method which remedy the above-mentioned disadvantages, including burnt deposits and a large time consumption.

In view of this object, the device for sterilization according to a first aspect of the invention is characterized in that at least one first nozzle is applied for spraying cold water onto the implements and at least one different nozzle is applied for spraying sterilizing fluid onto the implements.

This provides the advantage that both the cold water and the sterilizing fluid can begin to take effect immediately as the spraying begins. At commencement of the sterilization after the prerinsing, the hot water thus immediately has the right temperature of 82°C, as the nozzles applied and the pipe system have not been cooled by the cold water during the prerinsing and postrinsing in the preceding cycle. If a sterilizing fluid other than 82°C hot water is applied, this, too, will be able to take immediate effect, as the pipe system and the nozzles need not first be flushed. The same applies to the opposite shifts from hot water to cold water and from cold water to another sterilizing fluid. In both cases it thus becomes possible to realize a faster sterilization process. In this connection it should be noted that, in connection with the present invention, cold water means water of a temperature that is sufficiently low to ensure that proteins do not denature, for example a maximum temperature of 50°C.

In a preferred embodiment the sterilizing fluid is hot water, preferably water of a temperature of 82°C or higher. This has the advantage partly that the sterilizing fluid does not pollute, partly that it is readily available and does not require a special storage system.

In another preferred embodiment of the invention the sterilizing fluid comprises lactic acid. The use of lactic acid in an aqueous solution is approved, and furthermore it can be used cold, avoiding burnt deposits altogether here, too.

According to a preferred embodiment, the cold water has a temperature below 50°C, preferably around 35°C-45°C. In addition to the cooling of the blade, this provides good rinsing-off of blood and meat residues and thereby of proteins therefrom, so that burnt deposits are avoided, while water from one and the same source can be applied for the two rinsing operations before and after sterilization, respectively.

According to yet another embodiment of the invention, the cycle time is less than 10 s, preferably less than about 7 s. In that case, the cycle preferably comprises a first spraying with cold water for about 2 s, a spraying with sterilizing fluid for about 1 s, and a second spraying with cold water for about 2 s. Experiments have shown that this is sufficient partly to achieve the desired sterilization, partly to avoid the unwanted burnt deposits, while being well compatible with the production speed.

According to a particularly advantageous embodiment the method is initiated automatically by detection of the insertion of an implement in holding members in the sterilization device. At the said high production speed this has the advantage that the operator need only put down the implement, typically being the knife, whereupon the sterilization is executed automatically.

This is particularly advantageous in the case of a device for carrying out the method where two insertion openings are provided. In that case the operator can change between two implements simply by placing one implement in the device and taking the other one.

According to another aspect of the present invention, the device according to the invention is characterized in that the holding members are designed to grip the handle or haft of the implement to be sterilized. These holding members allow sterilization of both the blade and the transition between the blade and the handle of the knife. They also allow a change of implement without the operator having to concentrate much on placing the implement.

According to this aspect of the invention, it is ensured that the entire blade and parts of the handle are exposed and can be hit by the sterilizing fluid, which ensures good sterilization of the entire blade, particularly the upper parts thereof at the transition to the handle.

In a particularly preferred embodiment, the implement is a knife, and the holding member is adapted to grip the handle at a distance from the transition between the blade and handle of the knife. This further ensures the good sterilization mentioned above of the upper parts of the blade, but also of the lower part of the handle.

In yet another preferred embodiment, the holding members comprise parts at mutually adjustable distances. This means that they can be adjusted to the handles or hafts of different implements. This mainly means the handles of different knives, as the different operators that will alternately be using the sterilization device, will typically be using different knives. The holding members can thereby be adjusted to such different knives for optimum placing thereof in the sterilization device.

In a particularly advantageous embodiment, the holding members are adapted to allow passage of water or sterilizing fluid to the handle or haft of the implement to be sterilized, particularly to the transition between the handle and the blade, when the implement is placed in the holding members. This also allows good sterilization of the parts of the handle or haft which are closest to the blade and therefore also at the highest risk of getting into contact with the carcass being processed.

It is particularly preferred that the holding members comprise rolls with annular ribs. This minimizes the contact surface between the holding member and the handle or haft, while the grooves between the ribs allow the water and sterilizing fluid to pass to the handle or haft of the implement to be sterilized. Furthermore, the rolls allow the implement to position itself optimally in relation to the nozzles, i.e., so that the spraying with water and sterilizing fluid is exploited in the best possible way. Finally, when the implement to be sterilized is a knife, the rolls will only be able to affect its cutting ability to a negligible extent, as the edge of the blade, if it hits the roll, will make the roll turn. The edge does thus not cut the holding member and is therefore not blunted.

The invention will now be described in further detail on the basis of embodiments and the schematic drawing, in which
Fig. 1 shows a schematic perspective view of an embodiment of the invention with parts cut away,
Fig. 2 shows a diagram of the piping with a schematic illustration of the knife holder in a sectional view along the line II-II in Fig. 1, and
Fig. 3 shows a schematic sectional view of an alternative embodiment of the invention.

In the following description, the same reference numerals are used for like components of the embodiments described.

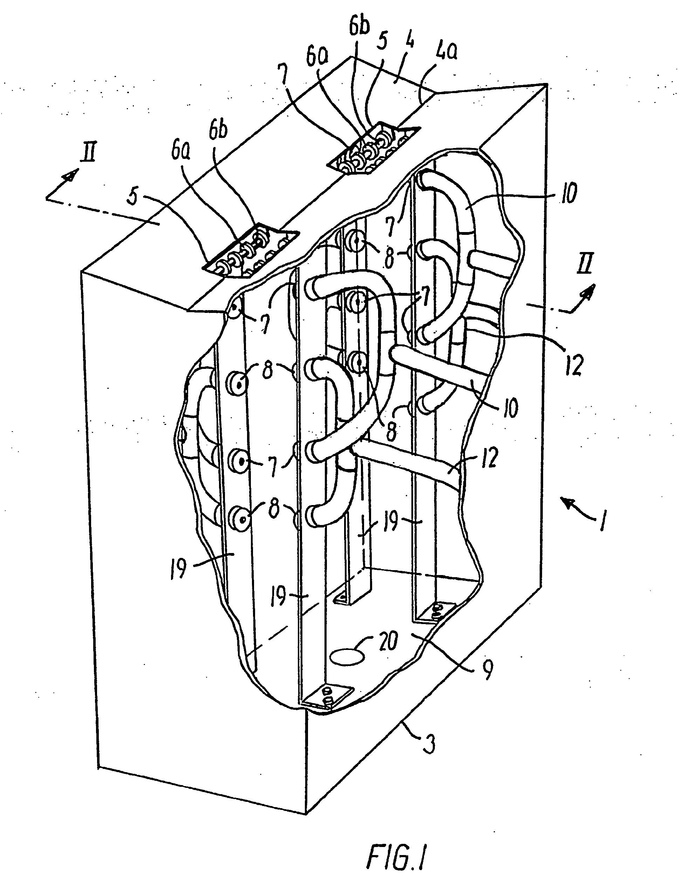

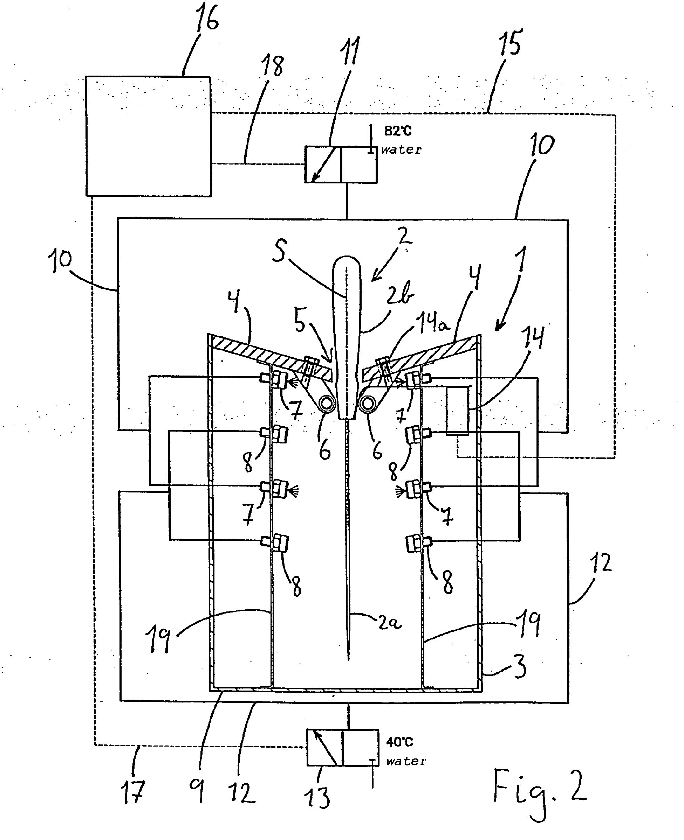

Fig. 1 is a schematic view of an embodiment of the sterilization device 1 according to the invention for sterilization of implements. Preferably, the device is intended for implements in the form of knives 2, as illustrated in Fig. 2. The sterilization device 1 comprises a substantially box-shaped housing 3. The primary function of the housing 3 is to limit splashes and squirts from the sterilization process, and therefore it need not be box-shaped. The housing 3 is preferably made of a material which is durable and easy to clean, for example corrosion-resistant steel or a suitable plastic material. At the top, the housing 3 is defined by an upper part 4, in which two insertion openings 5 are provided, through which the knives 2 to be sterilized can be inserted. Preferably, the upper part 4 of the housing 3 has a longitudinal, V-shaped recess 4a, at the bottom of which the insertion openings 5 are provided. The V-shaped recess 4a makes it easier to hit the insertion openings 5 with the knives 2 to be sterilized. The upper part 4 is preferably made of a material, for example plastic, which is sufficiently soft to avoid any impact on the cutting ability of the knife 2, if it misses the insertion opening 5.

Below the upper part 4, holding members 6 for the knives 2 to be sterilized are placed in connection with the insertion openings 5. For each of the two insertion openings 5, the holding members 6 preferably comprise two parallel rolls, placed at opposite sides of the respective insertion openings 5. The rolls preferably comprise a number of annular ribs 6a, defining between them a number of annular grooves 6b. These holding members 6 are adjustable so that the distance between the rolls can be varied, whereby knives 2 with different designs of the handle 2b can be retained. At the same time it ensures that the knives are retained somewhat up on the handle 2b by the ribs 6a on the rolls, so that not only the transition between the blade 2a and the handle 2b is exposed to access by cold water and sterilizing fluid, but also parts of the handle itself, which ensures good sterilization far up on the handle. Furthermore, the adjustability thereby allows the operator, who is to use the sterilization device 1, to set the sterilization device 1 to retain the exact size or type of knife he is using for ergonomic reasons, while maintaining good sterilization. The provision of such adjustability lies within the reach of a person skilled in the art and will therefore not be further described here.

As shown in Fig. 2 the adjustable holding members grip the handle 2b of the knife 2 inserted through the insertion opening 5. Due to the design of the holding members with the annular ribs 6a, the handle 2b of the knife 2 in question is substantially only touched at two points, one on either side. Thereby, not only the blade 2a to be sterilized, but also large parts of the handle 2b are exposed to the spraying with the sterilizing fluid and the cold water, respectively. This can take place by sterilizing fluid or water hitting the handle 2b directly, or by sterilizing fluid or water running down the handle 2b after impact. It therefore becomes possible to ensure good sterilization of the parts of the knife 2 that get into contact with the carcass being processed.

The opening 5 is slightly wider than the handle, but not more so than that the edges can serve to define the sideways movements of the knife, so that the blade is substantially kept in a symmetry plane S.

Like the upper part 4, the rolls are made of a material that does not affect the cutting ability of the knife, if touched directly by the edge. Moreover, the knife 2 will tend to slide off the rolls because they can turn, thus reducing the effect on the edge of the knife.

Fig. 2 further shows the arrangement of two rows of nozzles 7, 8. The two rows comprise both nozzles 7 for sterilizing fluid and nozzles 8 for cold water. The two rows of nozzles 7, 8 are arranged so that, when a knife 2 is placed in the holding members, they are located on either side of the blade 2a. Typically, it will be a symmetrical arrangement around a symmetry plane as indicated by S in Fig. 2. Figs. 1 and 2 show a schematic example of how the two rows of nozzles are provided by the nozzles in each row all being mounted in one and the same holder 19, which extends from the upper part 4 of the housing 3 to its bottom 9. Preferably, the same type of nozzles 7, 8 are arranged in each row at the same height above the bottom 9 of the housing 3, nozzles 7 for sterilizing fluid and nozzles 8 for cold water being arranged alternately within the row. In the example shown, each row has two nozzles 7 for sterilizing fluid and two nozzles 8 for cold water, but naturally other numbers can be chosen as long as it is ensured that the blade 2a is hit adequately by both sterilizing fluid and cold water. Other examples are three nozzles for sterilizing fluid and three for cold water, two for sterilizing fluid and three for cold water, three for sterilizing fluid and two for cold water, or any other suitable combination that ensures that the blade 2a is hit adequately by both sterilizing fluid and cold water.

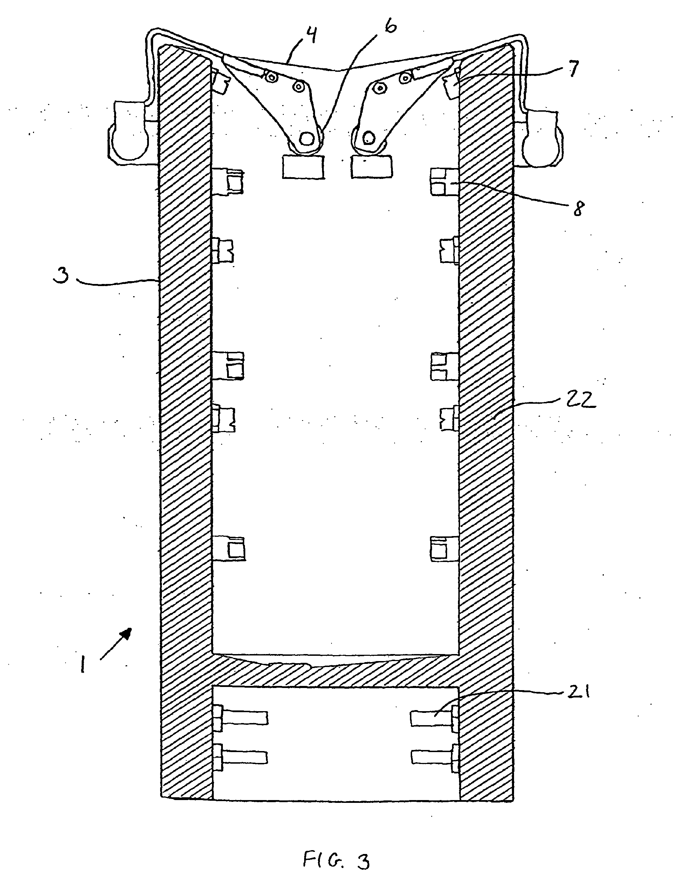

As it appears from the above, different nozzles 7 and 8 are used for sterilizing fluid and cold water, respectively. This should be understood so that one and the same nozzle 7, 8 can only be used for one and the same fluid, be it either sterilizing fluid or cold water. Although the nozzles 7, 8 according to the embodiment shown in Fig. 3 also have different structural designs, nothing prevents the different nozzles 7 and 8 from having identical designs. On the contrary, this would often be preferable as the viscosity and flow properties of sterilizing fluid and cold water, respectively, will be very similar, for example if the sterilizing fluid is hot water at 82°C, and the cold water has a temperature of about 40°C. In terms of design and storage, savings can be obtained by using only one type of nozzles.

The nozzles 7 and 8 may advantageously be adjusted so that they spray slightly downwards, for example 8° in relation to the horizontal plane as indicated in Fig. 2. This enhances the natural gravitational rinsing-off of sterilizing fluid and cold water with the impurities contained therein.

Having provided their effect, the sterilizing fluid and cold water consumed runs out of the housing through an outlet 20 at the bottom.

Different nozzles 7 and 8 are used for sterilizing fluid and cold water, respectively. As shown in Fig. 2 the different nozzles are supplied by separate systems. Thus, the nozzles 7 are connected to a supply 11 through conduits 10, which supply 11 delivers sterilizing fluid, illustrated in Fig. 2 with water at 82°C, but other sterilizing fluids may also be used. The other nozzles 8 are connected to a water supply for cold water through conduits 12, illustrated in Fig. 2 with water at 40°C, but other temperatures may also be used, for example 50°C or temperatures below 40°C.

In Fig. 2 the supply conduits 10 and 12 are shown purely schematically, while in Fig. 1 they are shown as flexible tubes. This is for illustration purposes only and a person skilled in the art will understand that other supply conduits can be used, such as pipes.

As shown in Fig. 3, according to an alternative embodiment such pipes can be integrated as ducts in the walls 22 of the housing 3, the nozzles 7, 8 being screwed directly into bores in the inner wall 22 of the housing 3, thus communicating with the ducts. In this embodiment the housing 3 is preferably made of a plastic material which, due to its low thermal conductivity and capacity, affects the temperatures of the water and the sterilizing fluid less than would be the case with corrosion-resistant steel, for example. The supply conduits 10 and 12 to the ducts in the walls 22 and the nozzles 7, 8 connected thereto may, for example, be passed into the device through a separate, subjacent housing and connected through T-pieces, preferably at the bottom of the device, to two sets of connecting pieces 21. The two sets of connecting pieces 21 are preferably located in respective inner walls 22 of the housing 3, so that the pieces communicate with respective ducts and thus communicate with the respective nozzles 7, 8 in the wall in question. This embodiment obviates the separate holding members 19 shown in Figs. 1 and 2 and the internal piping in the housing, so that the total external dimensions of the sterilization device 1 may be smaller.

Experiments have shown that, even with a supply of both sterilizing fluid and cold water to the nozzles 7, 8 in the respective systems at a relatively low pressure, for example 2-3 bar, the desired effects are achieved, i.e., both sufficient sterilization and prevention of burnt deposits.

In connection with each insertion opening 5, detection means 14 are provided for detection of the insertion of a knife 2 to be sterilized in the insertion opening 5 in question. In the embodiment shown in Fig. 2, the detection means are in the form of a sensor with a lever arm 14a, which can be actuated mechanically by the handle 2b, when a knife 2 is passed through the insertion opening 5 to the holding members 6. Alternatively, other detection means can be used, for example in the form of photocells or proximity detectors. Through a communication line 15, the detection means 14 transmit a signal to a control system 16, which initiates the sterilization cycle according to the invention, the different supplies 11, 13 for sterilizing fluid and cold water, respectively, being alternately actuated through communication lines 17, 18. These communication lines may for example be electric supply lines, which actuate and de-actuate magnetic valves, so that the nozzles 7, 8 are provided with the respective fluids through the supply conduits 10 and 12 due to the said pressure of 2 to 3 bar. The design of such control system 16 and the actuation of the supplies 11, 13 lie within the reach of a person skilled in the art and are not further described here.

The following is a description of a preferred embodiment of a sterilization cycle according to the invention, which the sterilization device 1 described above may be designed to carry out.

When a knife 2 to be sterilized is inserted through the insertion opening 5, its presence is detected by the detection means 14. A signal is then transmitted through the communication line 15 to the control unit 16, stating the presence of a knife 2 to be sterilized. The control unit 16 then actuates the supply 13 for cold water. This can be done for example in the form of a control signal via a communication line 17 to a magnetic valve. The cold water, which is under a pressure of 2 to 3 bar, then flows through the supply conduits 12 to the nozzles 8 connected thereto. Following a suitable period for rinsing-off of blood and meat residues from the blade 2a, the control device 16 again shuts off the supply 13 of cold water. Experiments have shown that a period of about 2 s is sufficient to ensure the rinsing-off. This applies particularly in the case of water at a temperature of about 40°C, which, as mentioned, means cold water in this context.

Then the control unit 16 actuates the supply 11 for sterilizing fluid, preferably water at a temperature of over 82°C. This can be done for example in the form of a control signal via a communication line 18 to a magnetic valve. The hot water, which is under a pressure of 2 to 3 bar, then flows through the supply conduits 10 to the nozzles 7 connected thereto. This situation is illustrated in Fig. 2. Following a suitable period to ensure sterilization of the blade 2a and the parts of the handle 2b that are hit by the hot water, the control device 16 again shuts off the supply of hot water. Experiments have shown that, since the hot water already has the requisite temperature to ensure sterilization, a period of about 1 s is sufficient to ensure the sterilization. This also applies if, according to an alternative embodiment of the method, lactic acid, preferably in an aqueous solution, is used as a sterilizing fluid instead of hot water, in which case the supply conduits for the nozzles need not first be flushed for water.

The control device 16 then re-actuates the supply 13 for cold water. The cold water then again flows through the supply conduits 12 to the nozzles 8 connected thereto. Following a suitable period for cooling of the blade 2a, the control device 16 again shuts off the supply 13 of cold water. Experiments have shown that a period of about 2 s is sufficient to ensure such cooling. In the case of the alternative embodiment, mentioned above, with lactic acid as sterilizing fluid, the period must be sufficient to remove all residues thereof from the blade 2a. Also in this case, a period of 2 s has proved suitable.

The three phases of the sterilization process, mentioned above, follow in immediate succession, and the entire sterilization process is thus completed within about 7 s, whereupon the knife 2 is ready for use again. The operator need only insert the next knife to be sterilized into the other insertion opening 5 and then grip the newly sterilized knife 2, which is ready for use. When the second knife 2 is inserted into the second insertion opening, the sterilization cycle begins at that point and is effected over the next 7 s, while the operator is using the first knife 2. The operator can thus comfortably change between the two knifes, which are used and sterilized alternately.

The method and device described above thus provide fast and efficient sterilization.

The invention is not limited to the embodiments described above. In the case where hot water is used as sterilizing fluid, the hot water for the nozzles can be provided in the device by arranging, for example, an electric heating coil at a suitable position between the water supply in the device and the nozzles for hot water. In that case the sterilization device requires only a supply of cold water.

As mentioned, the components of the sterilization device 1, such as the housing 3, may be made of any suitable material, including but not limited to corrosion-resistant steel or plastic.

Moreover, the sterilization device 1 may comprise a dividing plate, separating the two insertion openings 5 shown in Fig. 1 and extending down through the housing 3, so that two positioned knives are separated by the said dividing plate in substantially the entire height of the device, whereby fluid, etc., from the knife being sterilized cannot splash onto any second knife positioned in the second insertion opening 5 of the sterilization device 1.

It should be understood that the above embodiments are for illustration purposes only and thus may be modified in numerous ways within the scope of protection of the claims. As mentioned, particularly the adjustability of the holding members can be designed differently, but this applies correspondingly to the design of the nozzles, their position and the piping thereto. Furthermore, it should be noted that the holding members 6 and the separate nozzles described can also be used separately to achieve faster and safer sterilization of the implements than in prior art.

## Claims

1. A method for sterilization of implements, particularly knives (2), in a sterilization device (1), which applies a cycle comprising a spraying with cold water and a spraying with sterilizing fluid, the cold water and the sterilizing fluid being sprayed onto the implements through nozzles (7, 8), **characterized in that** different nozzles (7, 8) are used for a spraying with cold water and a spraying with sterilizing fluid.

2. A method according to claim 1, **characterized in that** the sterilizing fluid is hot water, preferably water of a temperature of 82°C or higher.

3. A method according to claim 1, **characterized in that** the sterilizing fluid comprises lactic acid.

4. A method according to any one of the preceding claims, **characterized in that** the cold water has a temperature below 50°C, preferably around 35°C - 45°C.

5. A method according to any one of the preceding claims, **characterized in that** the cycle time is less than 10 s, preferably less than about 7 s.

6. A method according to any of the preceding claims, **characterized in that** a cycle is used comprising a first spraying with cold water, a spraying with sterilizing fluid, and a second spraying with cold water.

7. A method according to claim 6, **characterized in that** the cycle comprises a first spraying with cold water for about 2 s, a spraying with sterilizing fluid for about 1 s, and a second spraying with cold water for about 2 s.

8. A method according to any one of the preceding claims, **characterized in that** it is initiated automatically by detection of the insertion of an implement in holding members in the sterilization device.

9. A device (1) for sterilization of implements, particularly knives (2), and comprising nozzles (7, 8) for spraying cold water and sterilizing fluid onto the implements, **characterized in that** at least one first nozzle (8) for spraying cold water onto the implements and at least one different second nozzle (7) for spraying sterilizing fluid onto the implements are provided.

10. A device (1) for sterilization of implements, particularly knives (2), and comprising a substantially closed housing (3) with an insertion opening (5) for at least one implement to be sterilized, holding members (6) for the implement to be sterilized being arranged in connection with the insertion opening (5), **characterized in that** the holding members (6) are designed to grip the handle (2b) or haft of the implement to be sterilized.

11. A device according to claim 10, **characterized in that** the implement is a knife (2), and that the holding member (6) is adapted to grip the handle at a distance from the transition between the blade (2a) and handle (2) of the knife.

12. A device (1) according to claim 10 or 11, **characterized in that** the holding members (6) comprise parts with a mutually adjustable distance.

13. A device (1) according to any one of claims 10 to 12, **characterized in that** the holding members (6) are designed to allow passage of water or sterilizing fluid to the handle (2b) or haft of the implement to be sterilized, particularly to the transition between the handle (2a) and the blade (2b), when the implement is placed in the holding members (6).

14. A device (1) according to any one of claims 10 to 13, **characterized in that** the holding members comprise rolls with annular ribs (6a).

15. A device (1) according to any one of claims 10 to 14, **characterized in that** two insertion openings (5) are provided.

16. A device (1) according to claim 9 or 10, **characterized in that** two pairs of nozzles (8) for cold water and two pairs of nozzles (7) for sterilizing fluid are provided, the nozzles in each pair being located in relation to an inserted implement so that they are on either side thereof.

17. A device (1) according to any one of claims 9 to 16, **characterized in that** it comprises means (14) for detecting the insertion of an implement to be sterilized and initiation of the sterilization.

18. A device (1) according to any one of claims 9 to 17, **characterized in that** the device (1) comprises at least one heating device, preferably at least one heating coil.
